# EUROPEAN PATENT APPLICATION

(11) **EP 4 813 377 A1**
(43) Date of publication of application: **30.09.2026**
(21) Application number: 24894669.1
(22) Date of filing: 21.11.2024
(51) Int. Cl.: A61K 31/00, A61P 35/00, A61P 35/04

(54) **PHARMACEUTICAL COMPOSITION FOR PREVENTING OR TREATING HEAD AND NECK CANCER**

(30) Priority: 21.11.2023 KR 20230162319; 20.11.2024 KR 20240166746
(71) Applicant: Astrion Inc., Seoul 02842 (KR)
(72) Inventor: JEONG, Geuk-Rae, Seoul 02842 (KR); PARK, Hee Jo, Seoul 02842 (KR)
(74) Representative: Dehns
(86) International application number: PCT/KR2024/018571
(87) International publication number: WO 2025/110778

(57) **Abstract**

The present invention relates to a use of a novel compound for the prevention, amelioration, or treatment of head and neck cancer. The novel compound of the present invention not only significantly inhibits the growth, proliferation, migration, and metastasis of head and neck cancer cells, but also simultaneously inhibits ANO1 and EGFR in head and neck cancer cells, thereby exerting a stronger anticancer effect through dual inhibition against these two proteins. In addition, when used in combination with an anticancer agent or the like, the compound can enhance the efficacy of the anticancer drugs and suppress drug resistance. Accordingly, the compound can be used as a dual-target anticancer agent for ANO1 and EGFR on its own, or as a combination agent with anticancer drugs, and is expected to be widely applicable in the field of prevention and treatment of head and neck cancer.

## Description

### [Technical Field]

The present invention relates to a use of a novel compound for the prevention, amelioration, or treatment of head and neck cancer.

This application claims priority to and the benefit of Korean Patent Application No. 10-2023-0162319, filed on November 21, 2023, and Korean Patent Application No. 10-2024-0166746, filed on November 20, 2024, the disclosure of which is incorporated herein by reference in its entirety.

### [Background Art]

Head and neck cancer, which affects the oral cavity, pharynx, and larynx, ranks sixth in incidence among cancers by organ type. Head and neck cancer is a common cancer, with 40,000 new cases occurring annually in the US alone and 600,000 worldwide. Although treatment outcomes have significantly improved recently due to advancements in diagnostic and therapeutic technologies, the five-year survival rate for advanced head and neck cancer does not exceed 50%, and approximately 300,000 patients worldwide die each year. The challenges in treating head and neck cancer include frequent local and distant metastasis. Moreover, when the cancer develops in or near the respiratory, vocal, and swallowing organs, various anatomical, anesthetic, and functional impairments are caused even after treatment. Due to the proximity of vital organs, securing sufficient resection margins during surgery is difficult, and consequently, chemotherapy/radiotherapy alone or concurrent chemoradiotherapy is commonly performed. Despite these efforts, the high recurrence rate remains a challenge in treatment. Therefore, there is an urgent need for the development of targeted therapies capable of effectively and selectively treating cancer cells while minimizing side effects.

In head and neck cancer, tumor cell-specific targeted therapies are expected to minimize toxicity and enhance therapeutic efficacy. Various therapeutic agents such as gene therapies and monoclonal antibodies (mAbs) are currently under development to target head and neck cancer cells. These targeted therapeutic agents are designed to exert their effects by targeting molecules characteristic of cancer cells and have been developed to act on various targets, including signal transduction pathways and angiogenesis.

More than 90% of head and neck cancer cases are histologically squamous cell carcinomas, and since the expression of epidermal growth factor receptor (EGFR/ErbB1/HER) is confirmed in approximately 90% of head and neck squamous cell carcinomas, EGFR may be considered the most studied molecular target in head and neck cancer. The high expression of EGFR is known to be associated with reduced survival rates, resistance to radiotherapy, local treatment failure, and increased distant metastasis. EGFR is a transmembrane protein cell surface receptor and a 170 kDa glycoprotein. EGFR is a member of the ErbB/HER family along with ErbB2 (HER2/neu), ErbB3 (HER3), and ErbB4 (HER4). EGFR ligands include epidermal growth factor (EGF) and transforming growth factor-α (TGF-α), and EGFR bound to these ligands exhibits tyrosine kinase activity, thereby increasing signaling pathways such as the mitogen-activated protein kinase (MAPK) pathway and phosphatidylinositol-3-kinase (PI3K)/AKT/mammalian target of rapamycin (mTOR) pathway, and playing an important role in cell growth, development, and differentiation.

Meanwhile, calcium-activated chloride channels (CaCCs) are widely expressed in various cells and tissues and serve to regulate physiological functions such as epithelial fluid secretion, smooth muscle contraction, sensory nerve signaling, and cell growth. Since anoctamin-1/transmembrane member 16 (AANO1/TMEM16A) was identified as a CaCC in 2008, various studies have shown that anoctamin-1 (ANO1) is highly expressed in the bronchi, intestines, glandular epithelium, smooth muscles, intestinal pacemaker cells, sensory nerves, and various types of tumor cells such as head and neck cancer, breast cancer, prostate cancer, and kidney cancer, and that ANO1 possesses diverse physiological functions. ANO1 is located on chromosome band 11q13 and is overexpressed in various cancer cells, such as head and neck cancer, breast cancer, and prostate cancer. Recent research has revealed that ANO1 is involved in cell growth, migration, cancerization, and cancer development. For example, in a mouse model, it was observed that inhibiting the expression of ANO1 in prostate cancer cells (PC3) suppressed cell growth, metastasis, invasion, and tumor development, and the ANO1 inhibitor T16Ainh-A01 suppressed the growth of interstitial cells of Cajal (ICC) and pancreatic cancer cells (CFPAC1) that express high levels of ANO1. Furthermore, it has been reported that inhibiting the expression of ANO1 in breast cancer cells suppresses cell growth, induces apoptosis, and inhibits tumor growth in a xenograft model. In addition, it has been found that inhibiting ANO1 exhibits an anti-cancer effect by inhibiting EGFR and calmodulin-dependent protein kinase 2 (CAMK2) signaling in head and neck cancer (head and neck squamous cell carcinoma (HNSCC)), esophageal squamous cell carcinoma (ESCC), and breast cancer cells. Therefore, recent research results suggest that pharmacological inhibition of ANO1 ion channel activity has potential as a treatment for head and neck cancer, esophageal cancer, gastrointestinal cancer, breast cancer, and prostate cancer that overexpress ANO1.

Thus, both ANO1 and EGFR proteins have the potential to be therapeutic targets for head and neck cancer. However, no substance capable of exhibiting excellent anti-cancer effects through the dual inhibition of ANO1 and EGFR has yet been discovered.

### [Disclosure]

### [Technical Problem]

The present invention relates to a use of a novel compound for the prevention, amelioration, or treatment of head and neck cancer, and was completed by confirming that the novel compound of the present invention inhibits the growth, proliferation, migration, and metastasis of head and neck cancer cells and simultaneously inhibits ANO1, which is a calcium-activated chloride channel, and EGFR, which is an epidermal growth factor receptor, thereby not only exerting an excellent anti-cancer effect but also exhibiting a high synergistic effect when administered in combination with an anti-cancer agent.

Therefore, an object of the present invention is to provide a pharmaceutical composition for preventing or treating head and neck cancer, comprising, as an active ingredient, a compound represented by the following Chemical Formula 1 or a pharmaceutically acceptable salt thereof.

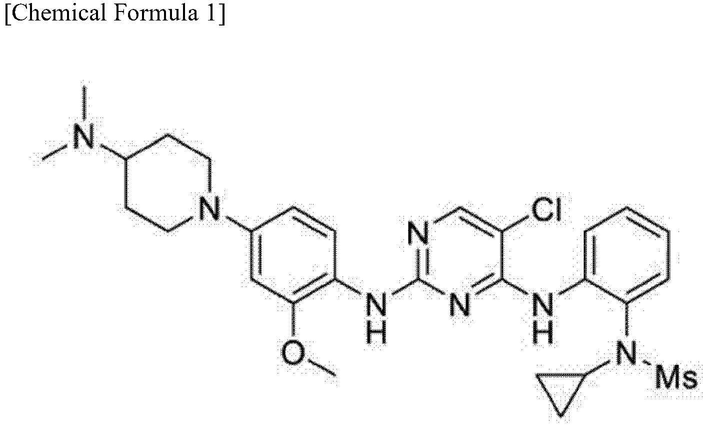

Another object of the present invention is to provide a pharmaceutical composition for preventing or treating head and neck cancer, comprising, as active ingredients, (a) the compound represented by Chemical Formula 1 or a pharmaceutically acceptable salt thereof; and (b) one or more anti-cancer agents selected from the group consisting of a taxane-based chemotherapy agent and a targeted anti-cancer agent against EGFR.

Yet another object of the present invention is to provide a pharmaceutical composition for enhancing an anti-cancer effect of an anti-cancer agent on head and neck cancer, comprising, as an active ingredient, the compound represented by Chemical Formula 1 or a pharmaceutically acceptable salt thereof.

However, technical problems to be solved in the present invention are not limited to the above-described problems, and other problems which are not described herein will be fully understood by those of ordinary skill in the art from the following descriptions.

### [Technical Solution]

In order to achieve the object, the present invention provides a pharmaceutical composition for preventing or treating head and neck cancer, comprising, as an active ingredient, a compound represented by the following Chemical Formula 1 or a pharmaceutically acceptable salt thereof.

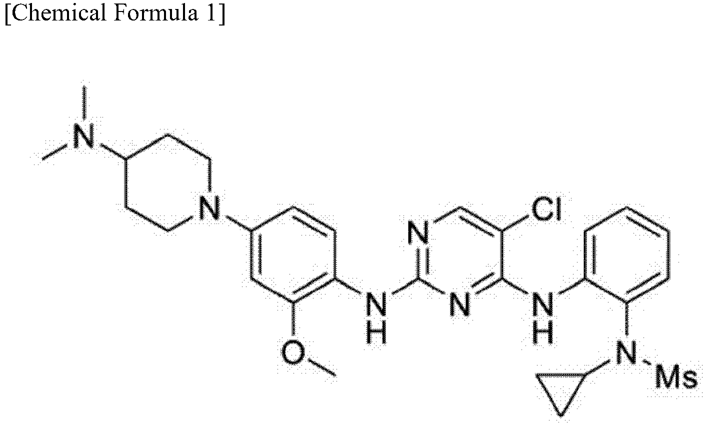

In one embodiment of the present invention, the head and neck cancer may be one or more selected from the group consisting of nasal cavity cancer, paranasal sinus cancer, oral cancer, nasopharyngeal cancer, oropharyngeal cancer, hypopharyngeal cancer, laryngeal cancer, cervical esophageal cancer, salivary gland cancer, salivary gland tumors, tongue cancer, thyroid cancer, and tonsil cancer, but is not limited thereto.

In another embodiment of the present invention, the compound or a pharmaceutically acceptable salt thereof may simultaneously inhibit EGFR and ANO1, but is not limited thereto.

In yet another embodiment of the present invention, the compound or a pharmaceutically acceptable salt thereof may satisfy one or more properties selected from the group consisting of the following, but is not limited thereto:
(a) inhibiting the proliferation or growth of cancer cells;
(b) inhibiting the migration of cancer cells;
(c) inhibiting the metastasis of cancer; and
(d) inhibiting the resistance of cancer to anti-cancer agents.

In yet another embodiment of the present invention, the anti-cancer agent may be one or more selected from the group consisting of a taxane-based chemotherapy agent and a targeted anti-cancer agent against EGFR, but is not limited thereto.

In yet another embodiment of the present invention, the taxane-based chemotherapy agent may be one or more selected from the group consisting of docetaxel, cabazitaxel, and paclitaxel, but is not limited thereto.

In yet another embodiment of the present invention, the targeted anti-cancer agent against EGFR may be one or more selected from the group consisting of cetuximab, panitumumab, osimertinib, gefitinib, afatinib, erlotinib, and lazertinib, but is not limited thereto.

In yet another embodiment of the present invention, the pharmaceutical composition may be administered in combination with a taxane-based chemotherapy agent or a targeted anti-cancer agent against EGFR, but is not limited thereto.

In yet another embodiment of the present invention, the pharmaceutical composition may be administered simultaneously, separately, or sequentially with the anti-cancer agent, but is not limited thereto.

Additionally, the present invention provides a pharmaceutical composition for preventing or treating head and neck cancer, comprising, as active ingredients, (a) the compound represented by Chemical Formula 1 or a pharmaceutically acceptable salt thereof; and (b) one or more anti-cancer agents selected from the group consisting of a taxane-based chemotherapy agent and a targeted anti-cancer agent against EGFR.

In one embodiment of the present invention, the compound or a pharmaceutically acceptable salt thereof may inhibit the resistance of cancer cells to the anti-cancer agent, but is not limited thereto.

In another embodiment of the present invention, the composition may be in the form of a mixture mixing the compound or a pharmaceutically acceptable salt thereof; and the anti-cancer agent, but is not limited thereto.

In yet another embodiment of the present invention, the composition may be in a form in which the compound or a pharmaceutically acceptable salt thereof; and the anti-cancer agent are each formulated to be administered simultaneously, separately, or sequentially, but is not limited thereto.

Additionally, the present invention provides a pharmaceutical composition for enhancing an anti-cancer effect of an anti-cancer agent on head and neck cancer, comprising, as an active ingredient, the compound represented by Chemical Formula 1 or a pharmaceutically acceptable salt thereof.

In one embodiment of the present invention, the anti-cancer agent may be one or more selected from the group consisting of a taxane-based chemotherapy agent and a targeted anti-cancer agent against EGFR, but is not limited thereto.

In another embodiment of the present invention, the taxane-based chemotherapy agent may be one or more selected from the group consisting of docetaxel, cabazitaxel, and paclitaxel, but is not limited thereto.

In yet another embodiment of the present invention, the targeted anti-cancer agent against EGFR may be one or more selected from the group consisting of cetuximab, panitumumab, osimertinib, gefitinib, afatinib, erlotinib, and lazertinib, but is not limited thereto.

In yet another embodiment of the present invention, the pharmaceutical composition may be administered simultaneously, separately, or sequentially with the anti-cancer agent, but is not limited thereto.

Additionally, the present invention provides a method of preventing, ameliorating, or treating head and neck cancer; or a method of enhancing an anti-cancer effect of an anti-cancer agent on head and neck cancer, comprising a step of administering the compound represented by Chemical Formula 1 or a pharmaceutically acceptable salt thereof; or a composition comprising the same, in a pharmaceutically effective amount to a subject in need thereof.

Additionally, the present invention provides a use of the compound represented by Chemical Formula 1 or a pharmaceutically acceptable salt thereof; or a composition comprising the same for preventing, ameliorating, or treating head and neck cancer; or for enhancing an anti-cancer effect of an anti-cancer agent on head and neck cancer.

Additionally, the present invention provides a use of the compound represented by Chemical Formula 1 or a pharmaceutically acceptable salt thereof; or a composition comprising the same for preparing a preparation for preventing, ameliorating, or treating head and neck cancer; or a preparation for enhancing an anti-cancer effect of an anti-cancer agent on head and neck cancer.

Additionally, the present invention provides a method of preventing, ameliorating, or treating head and neck cancer, comprising a step of administering (a) the compound represented by Chemical Formula 1 or a pharmaceutically acceptable salt thereof; and (b) one or more anti-cancer agents selected from the group consisting of a taxane-based chemotherapy agent and a targeted anti-cancer agent against EGFR; or a composition comprising the same, in a pharmaceutically effective amount to a subject in need thereof.

Additionally, the present invention provides a use of (a) the compound represented by Chemical Formula 1 or a pharmaceutically acceptable salt thereof; and (b) one or more anti-cancer agents selected from the group consisting of a taxane-based chemotherapy agent and a targeted anti-cancer agent against EGFR; or a composition comprising the same for preventing, ameliorating, or treating head and neck cancer.

Additionally, the present invention provides a use of (a) the compound represented by Chemical Formula 1 or a pharmaceutically acceptable salt thereof; and (b) one or more anti-cancer agents selected from the group consisting of a taxane-based chemotherapy agent and a targeted anti-cancer agent against EGFR; or a composition comprising the same for preparing a preparation for preventing, ameliorating, or treating head and neck cancer.

### [Advantageous Effects]

The novel compound of the present invention not only significantly inhibits the growth, proliferation, migration, and metastasis of head and neck cancer cells, but also may simultaneously inhibit ANO1 and EGFR in head and neck cancer cells, thereby exerting a stronger anti-cancer effect through dual inhibition of the two proteins. Additionally, when used in combination with an anti-cancer agent or the like, the compound may further enhance the efficacy of the anti-cancer agent and suppress cancer resistance. Therefore, the compound may be used as a dual-target anti-cancer agent for ANO1 and EGFR on its own, or may also be utilized as a combination preparation with an anti-cancer agent, and thus is expected to be widely utilized in the field of prevention and treatment of head and neck cancer.

### [Description of Drawings]

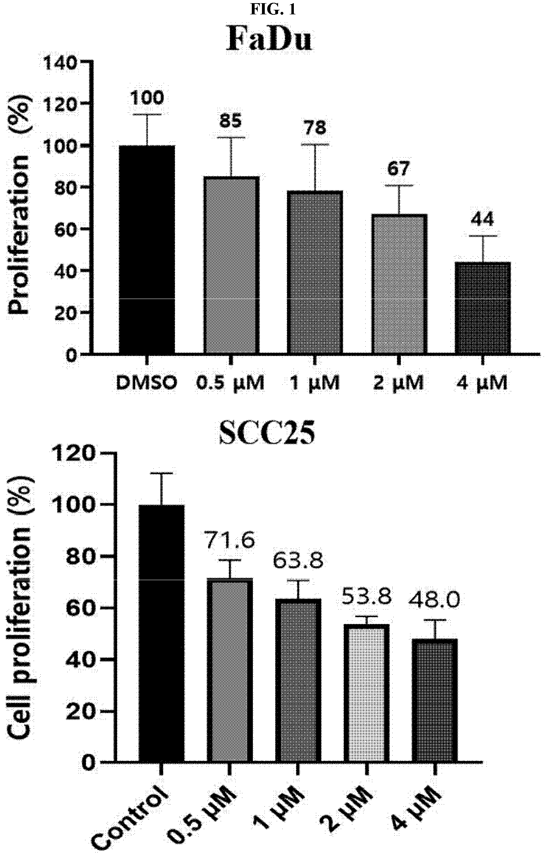

FIG. 1 shows the results of performing a CCK assay after treating human head and neck cancer cell lines FaDu cells and SCC-25 cells with compound AON-MG23 of the present invention at various concentrations in order to confirm the cell proliferation inhibitory effect of compound AON-MG23 of the present invention on human head and neck cancer cells.

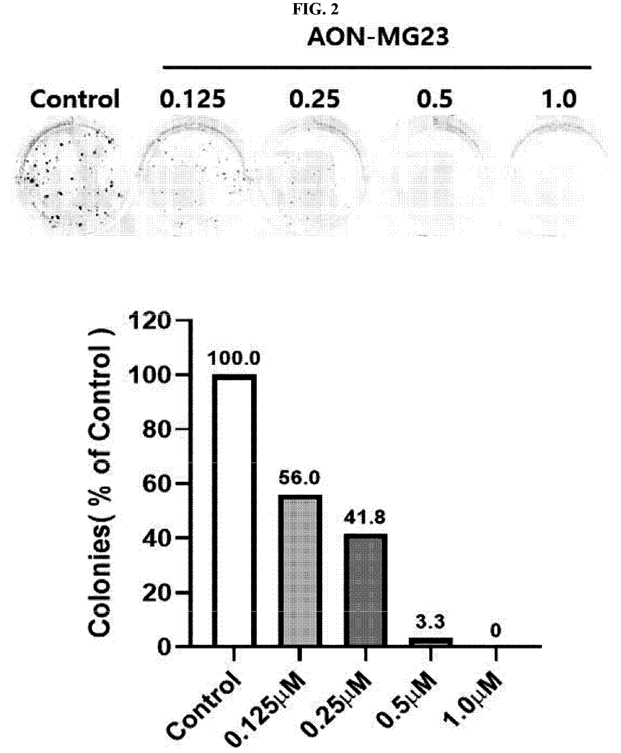

FIG. 2 shows the results of photographing and analyzing human head and neck cancer cell line FaDu cells after treating the cells with compound AON-MG23 of the present invention at various concentrations in order to confirm the effect of compound AON-MG23 of the present invention on the clonogenicity of head and neck cancer cells.

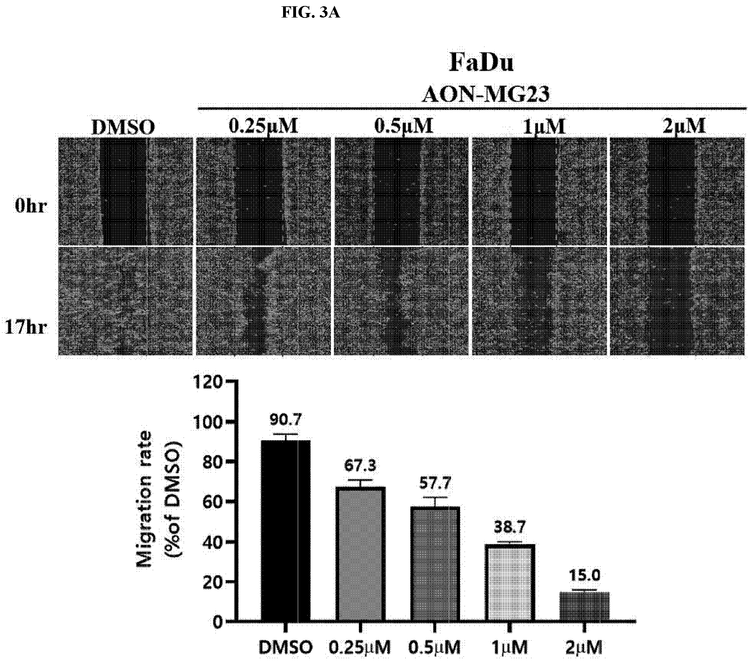

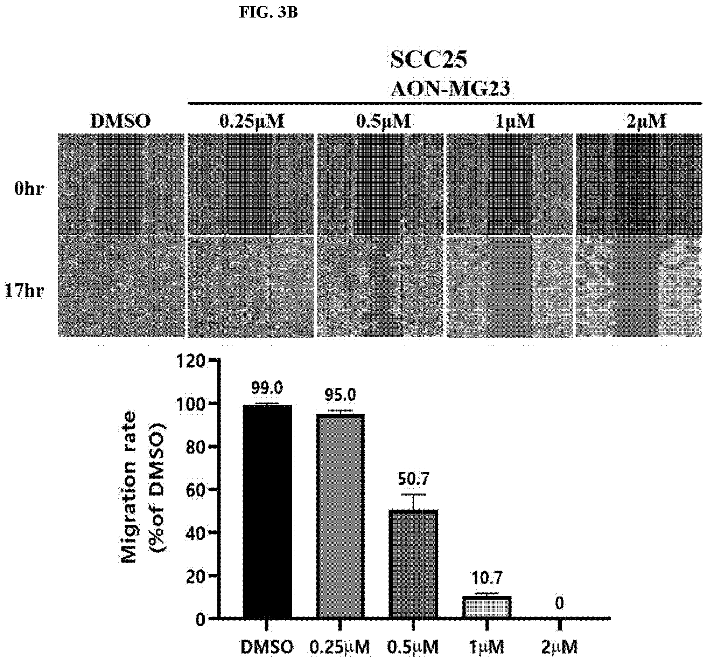

FIGS. 3A and 3B show the results of photographing and analyzing the degree of cell migration over time after treating human head and neck cancer cell lines FaDu cells and SCC-25 cells with compound AON-MG23 of the present invention at various concentrations in order to confirm the inhibitory effect of compound AON-MG23 of the present invention on cell migration in human head and neck cancer cells.

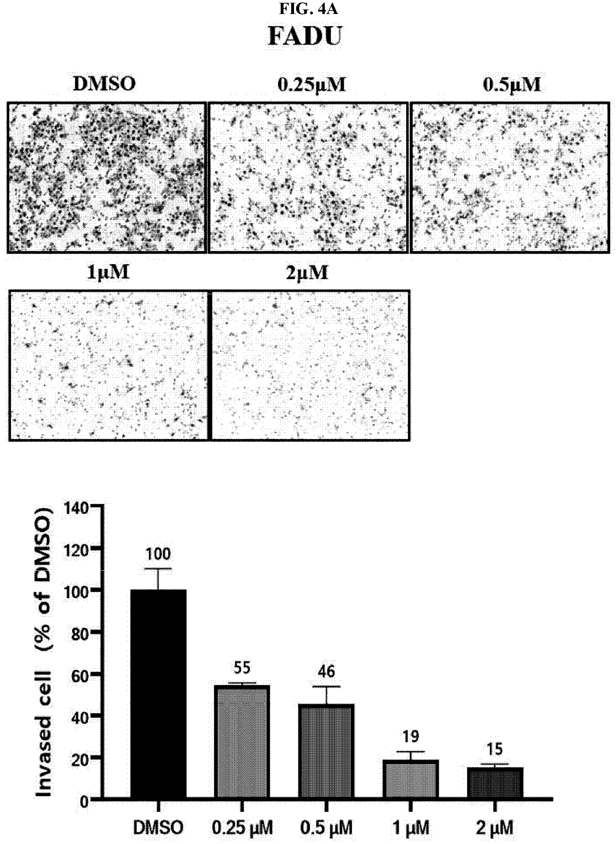

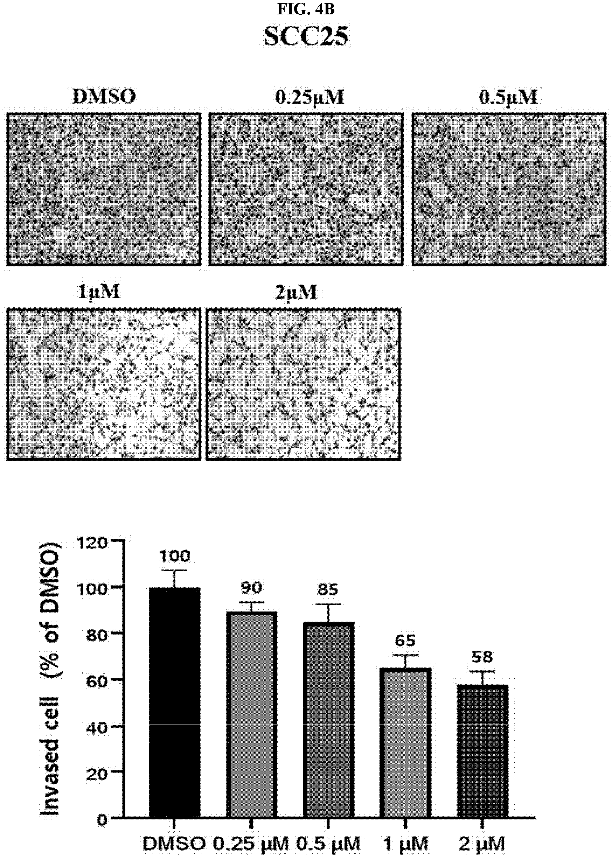

FIGS. 4A and 4B show the results of photographing and analyzing the degree of penetration of cells into Matrigel after treating human head and neck cancer cell lines FaDu cells and SCC-25 cells cultured on Matrigel with compound AON-MG23 of the present invention at various concentrations in order to confirm the inhibitory effect of compound AON-MG23 of the present invention on head and neck cancer cell metastasis.

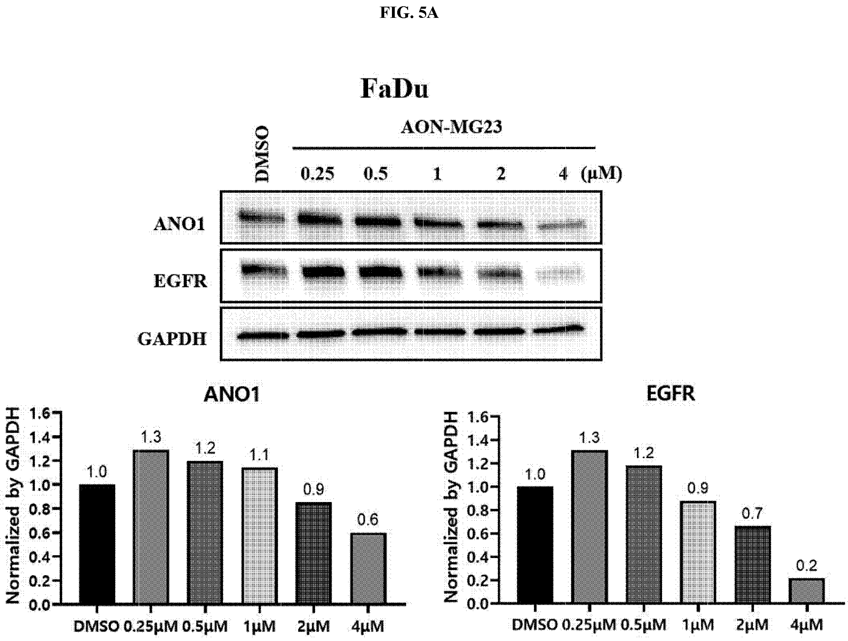

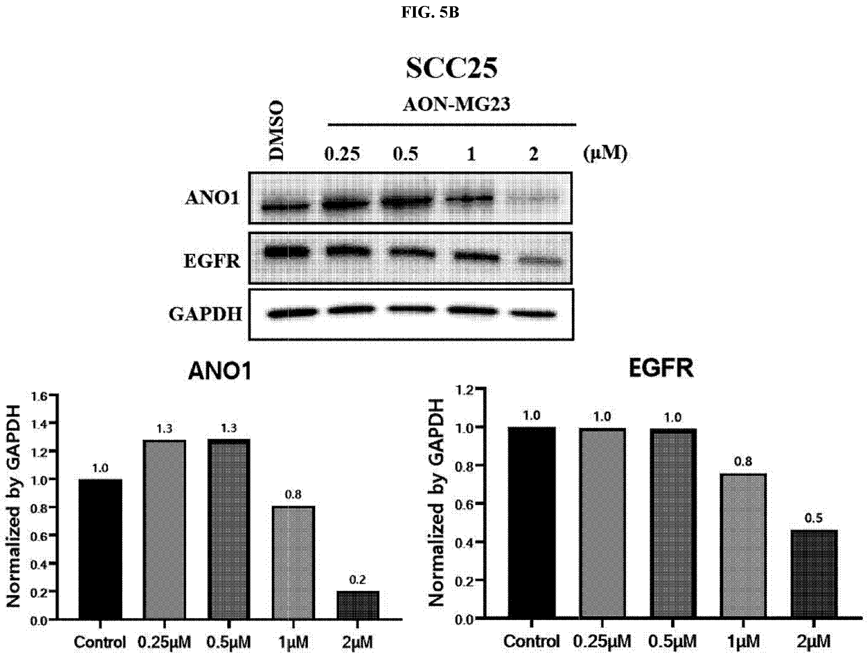

FIGS. 5A and 5B show the results of performing Western blot after treating human head and neck cancer cell lines FaDu cells and SCC-25 cells with compound AON-MG23 of the present invention at various concentrations in order to confirm the effect of compound AON-MG23 of the present invention on the expression of EGFR and ANO1 proteins in head and neck cancer cells, wherein the upper panels are images of protein bands detected by Western blot, and the lower panels quantify EGFR and ANO1 protein levels.

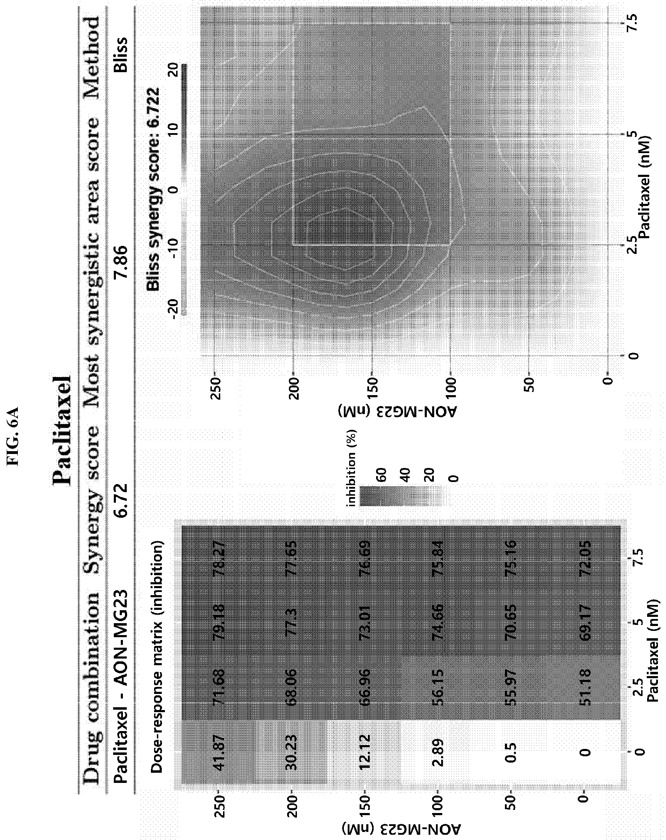

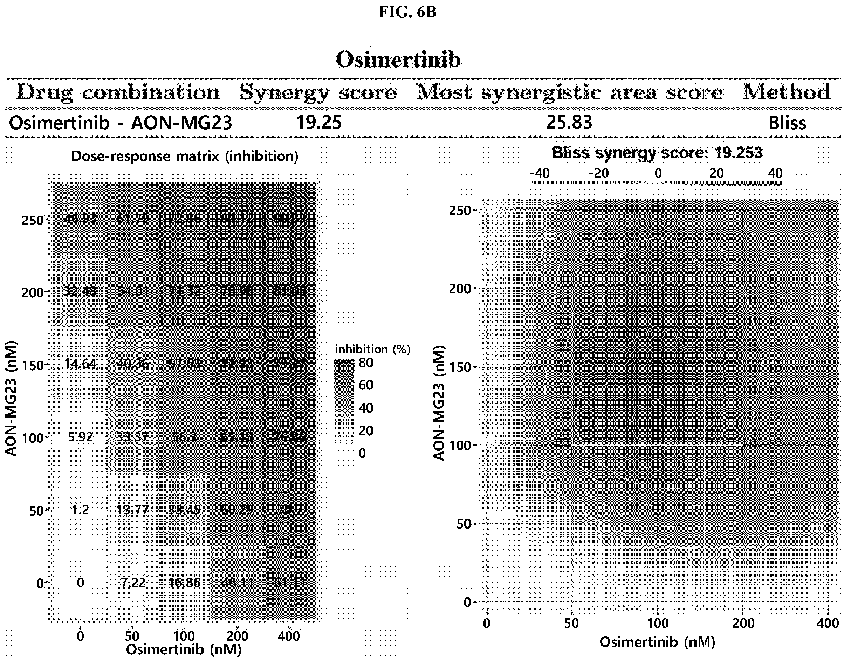

FIGS. 6A and 6B show the results of analysis after treating human head and neck cancer cell line FaDu cells with compound AON-MG23 of the present invention and paclitaxel (FIG. 6A) or osimertinib (FIG. 6B) at various concentrations in order to confirm the combination effect of compound AON-MG23 of the present invention with an anti-cancer agent in head and neck cancer cells.

### [Best Mode]

The present inventors completed the present invention by confirming that the novel compound of the present invention inhibits the growth, proliferation, metastasis, and migration of head and neck cancer cells and simultaneously inhibits ANO1, which is a calcium-activated chloride channel, and EGFR, which is an epidermal growth factor receptor, thereby not only exerting an excellent anti-cancer effect but also exhibiting a high synergistic effect when administered in combination with paclitaxel, which is a chemotherapy agent, or osimertinib, which is a targeted anti-cancer agent against EGFR.

Hereinafter, the present invention will be described in detail.

The present invention provides a pharmaceutical composition for preventing or treating head and neck cancer, comprising, as an active ingredient, a compound represented by the following Chemical Formula 1 or a pharmaceutically acceptable salt thereof.

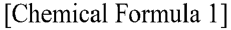

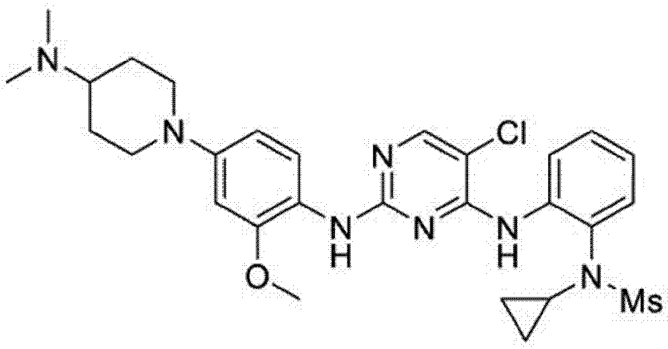

The compound represented by Chemical Formula 1 may be referred to as "N-(2-((5-chloro-2-((4-(4-(dimethylamino)piperidin-1-yl)-2-methoxyphenyl)amino)pyrimidin-4-yl)amino)phenyl)-N-cyclopropylmethanesulfonamide" or "AON-MG23," and "Ms" in Chemical Formula 1 means methylsulfonyl, that is, -SO₂(CH₃).

Hereinafter, unless otherwise specified, the terms "compound of the present invention" or "compound represented by Chemical Formula 1" and the like are used as concepts including the compound represented by Chemical Formula 1 itself, salts thereof, isomers thereof, and the like.

As used herein, the term "pharmaceutically acceptable salt" includes all salts derived from pharmaceutically acceptable inorganic acids, organic acids, or bases.

As used herein, the term "pharmaceutically acceptable" refers to a compound or composition that is suitable for use in contact with the tissue of a subject (for example, a human) within the scope of sound medical judgment, with a reasonable benefit/risk ratio, without excessive toxicity, irritation, allergic response, or other problems or complications.

Examples of suitable acids include hydrochloric acid, bromic acid, sulfuric acid, nitric acid, perchloric acid, hydroiodic acid, fumaric acid, maleic acid, phosphoric acid, glycolic acid, lactic acid, salicylic acid, succinic acid, toluene-p-sulfonic acid, tartaric acid, (+)-L-tartaric acid, di-L-tartaric acid, acetic acid, trichloroacetic acid or trifluoroacetic acid, 2,2-dichloroacetic acid, acylated amino acids, adipic acid, alginic acid, ascorbic acid, L-aspartic acid, 4-acetamidobenzoic acid, (+)-camphoric acid, camphorsulfonic acid, (+)-(1S)-camphorsulfonic acid, capric acid, caproic acid, caprylic acid, cinnamic acid, cyclamic acid, dodecylsulfuric acid, ethane-1,2-disulfonic acid, ethanesulfonic acid, 2-hydroxyethanesulfonic acid, galactaric acid, gentisic acid, glucoheptonic acid, D-gluconic acid, D-glucuronic acid, L-glutamic acid, α-oxo-glutaric acid, hippuric acid, (+)-L-lactic acid, (+-)-DL-lactic acid, lactobionic acid, (-)-L-malic acid, (+-)-DL-mandelic acid, citric acid, methanesulfonic acid, formic acid, benzoic acid, malonic acid, gluconic acid, naphthalene-2-sulfonic acid, naphthalene-1,5-disulfonic acid, benzenesulfonic acid, 1-hydroxy-2-naphthoic acid, nicotinic acid, oleic acid, orotic acid, oxalic acid, di-oxalic acid, palmitic acid, pamoic acid, L-pyroglutamic acid, salicylic acid, 4-amino-salicylic acid, sebacic acid, stearic acid, tannic acid, thiocyanic acid, camsylic acid, undecylic acid, and the like. An acid addition salt may be prepared by a conventional method, for example, by dissolving the compound in an excess aqueous acid solution and precipitating the salt using a water-miscible organic solvent such as methanol, ethanol, acetone, or acetonitrile. Additionally, the acid addition salt may be prepared by heating equimolar amounts of the compound and an acid in water or alcohol and then evaporating the mixture to dryness, or by suction-filtering the precipitated salt.

The scope of the compound of the present invention may include not only pharmaceutically acceptable salts, but also all isomers, hydrates, and solvates that may be prepared by conventional methods.

In the present invention, the term "isomer" refers to a compound that has the same molecular formula but differs in the connection manner or spatial arrangement of constituent atoms within the molecule. The isomers include, for example, structural isomers and stereoisomers. The stereoisomer may be a diastereomer or an enantiomer. An enantiomer refers to an isomer that is not superimposable on its mirror image, like the relationship between the left hand and the right hand, and is also referred to as an optical isomer. Enantiomers are classified as R (Rectus: clockwise) and S (Sinister: counterclockwise) when four or more substituents on a chiral center carbon are different from each other. A diastereomer refers to a stereoisomer that is not in a mirror-image relationship, and may be classified into cis-trans isomers generated by differences in the spatial arrangement of atoms.

The compound of the present invention is characterized by exerting an excellent anti-cancer effect by simultaneously inhibiting the expression or activity of EGFR and ANO1. The effect of preventing and/or treating head and neck cancer includes not only an effect of inhibiting the growth of head and neck cancer cells, but also an effect of inhibiting the progression or worsening of head and neck cancer caused by migration, invasion, metastasis, and the like. In the present invention, "EGFR (epidermal growth factor receptor)" is a transmembrane glycoprotein that is a member of the protein kinase superfamily and is a receptor for epidermal growth factor family proteins. EGFR is a major regulator of cell growth, and when epidermal growth factor (EGF), which is a ligand, binds to EGFR, the receptor undergoes dimerization and tyrosine autophosphorylation and induces cell proliferation. Overexpression (amplification) or hyperactivity of EGFR is frequently observed in various cancers such as anal cancer, head and neck cancer, and glioblastoma, and somatic mutations associated with EGFR cause continuous activation of EGFR, thereby inducing uncontrolled cell division. In particular, EGFR gene rearrangement and EGFR gene amplification are patterns observed in various cancers.

In the present invention, "Anoctamin 1 (ANO1)" is a calcium-activated chloride ion channel (Cl- channel), which is also referred to as "transmembrane protein 16A (TMEM16A)," and ANO1 is expressed in various normal cells including epithelial cells, smooth muscle cells, vascular endothelial cells, neurons, and the like, and performs various physiological roles by regulating fluid secretion, muscle contraction, pain sensation, and the like. However, ANO1 is known to be involved in cancerization of cells and cancer development. In fact, overexpression of ANO1 promotes signaling pathways that stimulate proliferation and migration in cancer cells, and inhibition of ANO1 has been reported to inhibit migration and growth of cancer cells. High expression of ANO1 appears to be associated with high expression of EGFR and STAT3, and inhibition of ANO1 is also known to improve the response of head and neck squamous cell carcinoma to EGFR/HER2 targeted therapy. Therefore, ANO1 has attracted attention as a target for cancer treatment, but an ANO1 inhibitor that actually exhibits an excellent anti-cancer effect has not yet been discovered. The compound of the present invention effectively inhibits the expression of ANO1 in head and neck cancer cells and has been confirmed to inhibit migration, proliferation, metastasis, and the like of head and neck cancer cells, and thus may be used for the prevention, treatment, and inhibition of metastasis of head and neck cancer. Furthermore, previous studies on the improvement of the effect of EGFR/HER2 targeted therapy by ANO1 inhibition suggest that the compound of the present invention may increase the responsiveness of head and neck cancer cells to EGFR/HER targeted therapy through the ANO1 inhibitory effect and inhibit the development of resistance.

In particular, the compound according to the present invention simultaneously inhibits the expression of EGFR together with ANO1 in head and neck cancer cells, and thus may exert a stronger anti-cancer effect through simultaneous inhibition of ANO1 and EGFR. The association between ANO1 and EGFR is well known, and upregulation of ANO1 is associated with the signaling pathway of EGFR and has been reported to have a significant effect on remodeling of the phosphorylated proteome after epidermal growth factor (EGF) stimulation. Additionally, in cancer cells, ANO1 may form a functional complex with EGFR and jointly regulate cell proliferation. Therefore, when EGFR and ANO1 are simultaneously inhibited using the compound of the present invention, the growth of head and neck cancer may be more effectively inhibited.

Additionally, ANO1 is known to be overexpressed in cancer cells and to contribute to migration of cancer cells and tumor metastasis, and signaling by EGFR is also known to create a tumor microenvironment that facilitates tumor metastasis. Therefore, the compound of the present invention may effectively inhibit metastasis of head and neck cancer through simultaneous inhibition of ANO1 and EGFR. The term "metastasis" refers to a state in which a malignant tumor has spread to another tissue away from the organ in which the malignant tumor occurred. Therefore, the composition according to the present invention may prevent and treat head and neck cancer from spreading throughout the body by inhibiting metastasis of head and neck cancer.

In the present invention, the term "tumor" is used interchangeably with "cancer" and typically refers to a condition characterized by uncontrolled cell growth, migration, and proliferation. The cancer according to the present invention is head and neck cancer, includes primary and recurrent cancer, and includes both benign and malignant tumors. The head and neck cancer may include all tumors occurring in the face, nose, throat, oral cavity, larynx, pharynx, salivary glands, and thyroid, except for tumors occurring in the brain and eyeballs, and in the present invention, the head and neck cancer may be one or more selected from the group consisting of nasal cavity cancer, paranasal sinus cancer, oral cancer, nasopharyngeal cancer, oropharyngeal cancer, hypopharyngeal cancer, laryngeal cancer, cervical esophageal cancer, salivary gland cancer, salivary gland tumors, tongue cancer, thyroid cancer, and tonsil cancer, but is not limited thereto. According to one example of the present invention, the head and neck cancer may be hypopharyngeal cancer and/or tongue cancer, preferably hypopharyngeal squamous cell carcinoma (FaDu cells) and/or tongue squamous cell carcinoma (SCC-25 cells).

In particular, the head and neck cancer according to the present invention may be head and neck cancer expected to be ameliorated or treated by simultaneously inhibiting ANO1 and EGFR. Preferably, the head and neck cancer according to the present invention may be associated with one or more mutations selected from the group consisting of EGFR (epidermal growth factor receptor) and ANO1 (Anoctamin 1). That is, the cancer according to the present invention may be head and neck cancer having a mutation in EGFR and/or ANO1. The mutation in EGFR and/or ANO1 includes amplification of the EGFR and/or ANO1 gene, overexpression of the protein, hyperactivity of the protein, continuous activation of the protein, and the like. That is, the head and neck cancer according to the present invention may have higher expression or activity of EGFR and/or ANO1 compared to normal cells. Alternatively, the head and neck cancer according to the present invention may be one in which the activated state of EGFR and/or ANO1 is maintained. Since the compound according to the present invention may simultaneously inhibit the expression and activity of ANO1 and EGFR, the compound may exert a particularly excellent anti-cancer effect against head and neck cancer accompanied by a mutation in EGFR and/or ANO1.

In the present invention, the compound or a pharmaceutically acceptable salt thereof may simultaneously inhibit EGFR and ANO1, but is not limited thereto.

In the present invention, the compound or a pharmaceutically acceptable salt thereof may satisfy one or more properties selected from the group consisting of the following, but is not limited thereto:
(a) inhibiting the proliferation or growth of cancer cells;
(b) inhibiting the migration of cancer cells;
(c) inhibiting the metastasis of cancer; and
(d) inhibiting the resistance of cancer to anti-cancer agents.

In the present invention, the anti-cancer agent in (d) may be one or more selected from the group consisting of a taxane-based chemotherapy agent and a targeted anti-cancer agent against EGFR, but is not limited thereto.

The present inventors confirmed through specific examples that head and neck cancer cells treated with the compound according to the present invention exhibited inhibited growth, proliferation, migration, and metastasis (invasiveness). Furthermore, the present inventors confirmed that the compound of the present invention may simultaneously inhibit ANO1 and EGFR, that is, dual inhibition, and therefore, the compound of the present invention may exert a more excellent anti-cancer effect compared to a single inhibitor of ANO1 or EGFR, and in particular, may inhibit the development of resistance of cancer cells to an anti-cancer agent through ANO1 inhibition and enhance the anti-cancer effect of the anti-cancer agent. For example, the present inventors confirmed through experiments that the compound of the present invention exhibits a high synergistic effect when administered in combination with paclitaxel, which is a taxane-based chemotherapy agent, or osimertinib, which is an EGFR inhibitor (targeted anti-cancer agent).

Therefore, in the present invention, the pharmaceutical composition may be administered in combination with a taxane-based chemotherapy agent or a targeted anti-cancer agent against EGFR, and the pharmaceutical composition may be administered simultaneously, separately, or sequentially with the anti-cancer agent, but is not limited thereto.

In the present invention, the term "chemotherapy agent" collectively refers to agents that exhibit anti-cancer activity, that is, cytotoxicity against cancer cells, mainly by directly acting on DNA to block DNA replication, transcription, and translation processes, or by interfering with the synthesis of nucleic acid precursors in metabolic pathways and inhibiting cell division, and the term "taxane-based chemotherapy agent" refers to a chemotherapy agent that inhibits proliferation by interfering with cell division of cancer cells by disrupting the process by which microtubules, which are cellular organelles involved in division and self-replication during cell division, are separated. The taxane-based chemotherapy agent may be one or more selected from the group consisting of docetaxel, cabazitaxel, and paclitaxel, but is not limited thereto.

In the present invention, the term "targeted anti-cancer agent" refers to an agent that exhibits an anti-cancer effect by targeting a protein or gene specifically altered in cancer cells or cancer tissue and interfering with molecular activity involved in the growth and development of cancer. The targeted anti-cancer agent against EGFR refers to an agent that exhibits an anti-cancer effect by targeting EGFR and interfering with molecular activity involved in the growth and development of cancer, and the targeted anti-cancer agent against EGFR may be one or more selected from the group consisting of cetuximab, panitumumab, osimertinib, gefitinib, afatinib, erlotinib, and lazertinib, but is not limited thereto.

Additionally, the compound according to the present invention may enhance the anti-cancer effect of an anti-cancer agent by increasing the activity of the anti-cancer agent against head and neck cancer or increasing the responsiveness of cancer cells to the anti-cancer agent. Enhancement of the responsiveness of cancer cells to the anti-cancer agent means that inhibition of growth, inhibition of proliferation, inhibition of migration, inhibition of metastasis, and the like of cancer cells by the anti-cancer agent are further enhanced.

In the present invention, "enhancing an anti-cancer effect" refers to all effects that may consequently enhance the function of an anti-cancer agent, and is a concept including not only enhancing the anti-cancer effect of the anti-cancer agent, such as inhibition of cancer cell growth, inhibition of cancer metastasis, and inhibition of cancer recurrence, but also consequently enhancing the anti-cancer effect by inhibiting the formation of resistance or tolerance of cancer cells to the anti-cancer agent.

Therefore, the compound according to the present invention may be used as a compound for combination administration with a known anti-cancer agent for the purpose of enhancing the anti-cancer effect of the anti-cancer agent.

For example, the present invention provides a pharmaceutical composition for preventing or treating head and neck cancer, comprising, as active ingredients, (a) the compound represented by Chemical Formula 1 or a pharmaceutically acceptable salt thereof; and (b) one or more anti-cancer agents selected from the group consisting of a taxane-based chemotherapy agent and a targeted anti-cancer agent against EGFR.

In the present invention, the compound or a pharmaceutically acceptable salt thereof may inhibit the resistance of cancer cells to the anti-cancer agent, but is not limited thereto.

In the present invention, the composition may be in the form of a mixture in which the compound or a pharmaceutically acceptable salt thereof and an anti-cancer agent are mixed, and may be in a form for simultaneous administration of the compound or a pharmaceutically acceptable salt thereof and the anti-cancer agent.

In the present invention, the composition may be in a form in which the compound or a pharmaceutically acceptable salt thereof and the anti-cancer agent are each formulated to be administered simultaneously, separately, or sequentially. In this case, the composition may be a pharmaceutical composition for combination administration for simultaneous or sequential administration, comprising a first pharmaceutical composition comprising, as an active ingredient, the compound or a salt thereof in a pharmaceutically effective amount; and a second pharmaceutical composition comprising, as an active ingredient, the anti-cancer agent in a pharmaceutically effective amount. At this time, in the case of sequential administration, the order of administration is not limited, and the administration regimen may be appropriately adjusted according to the condition of the patient and the like. That is, when the pharmaceutical composition is a pharmaceutical composition for combination administration for sequential administration, the composition may be one in which the compound or a salt thereof ("first component") is first administered and then the anti-cancer agent ("second component") is administered, and the reverse order is also possible.

Additionally, since the compound of the present invention may enhance the anti-cancer effect of an anti-cancer agent by increasing the activity of the anti-cancer agent against head and neck cancer or increasing the responsiveness of cancer cells to the anti-cancer agent, the present invention may provide a pharmaceutical composition for enhancing an anti-cancer effect of an anti-cancer agent on head and neck cancer, comprising, as an active ingredient, the compound represented by Chemical Formula 1 or a pharmaceutically acceptable salt thereof.

In the present invention, the anti-cancer agent may be one or more selected from the group consisting of a taxane-based chemotherapy agent and a targeted anti-cancer agent against EGFR, but is not limited thereto.

In the present invention, the taxane-based chemotherapy agent may be one or more selected from the group consisting of docetaxel, cabazitaxel, and paclitaxel, and according to one example of the present invention, the taxane-based chemotherapy agent may be paclitaxel, but is not limited thereto.

In the present invention, the targeted anti-cancer agent against EGFR may be one or more selected from the group consisting of cetuximab, panitumumab, osimertinib, gefitinib, afatinib, erlotinib, and lazertinib, and according to one example of the present invention, the targeted anti-cancer agent against EGFR may be osimertinib, but is not limited thereto.

In the present invention, the pharmaceutical composition may be administered simultaneously, separately, or sequentially with the anti-cancer agent, and the order of administration is not limited, and the administration regimen may be appropriately adjusted according to the type of cancer, the type of anti-cancer agent, the condition of the patient, and the like.

The content of the compound in the composition of the present invention may be appropriately adjusted depending on the symptoms of the disease, the degree of progression of the symptoms, the condition of the patient, and the like, and may be, for example, 0.0001 to 99.9% by weight, or 0.001 to 50% by weight, based on the total weight of the composition, but is not limited thereto. The content ratio is a value based on the dry weight after removing the solvent.

The pharmaceutical composition according to the present invention may further include a suitable carrier, excipient, and diluent which are commonly used in the preparation of pharmaceutical compositions. The excipient may be, for example, one or more selected from the group consisting of a diluent, a binder, a disintegrant, a lubricant, an adsorbent, a humectant, a film-coating material, and a controlled release additive.

The pharmaceutical composition according to the present invention may be used by being formulated, according to commonly used methods, into a form such as powders, granules, sustained-release-type granules, enteric granules, liquids, eye drops, elixirs, emulsions, suspensions, spirits, troches, aromatic water, lemonades, tablets, sustained-release-type tablets, enteric tablets, sublingual tablets, hard capsules, soft capsules, sustained-release-type capsules, enteric capsules, pills, tinctures, soft extracts, dry extracts, fluid extracts, injections, capsules, perfusates, or a preparation for external use, such as plasters, lotions, pastes, sprays, inhalants, patches, sterile injectable solutions, or aerosols. The preparation for external use may have a formulation such as creams, gels, patches, sprays, ointments, plasters, lotions, liniments, pastes, or cataplasmas.

As the carrier, the excipient, and the diluent that may be included in the pharmaceutical composition according to the present invention, lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil may be used.

For formulation, commonly used diluents or excipients such as fillers, thickeners, binders, wetting agents, disintegrants, and surfactants are used.

As additives of tablets, powders, granules, capsules, pills, and troches according to the present invention, excipients such as corn starch, potato starch, wheat starch, lactose, white sugar, glucose, fructose, D-mannitol, precipitated calcium carbonate, synthetic aluminum silicate, dibasic calcium phosphate, calcium sulfate, sodium chloride, sodium hydrogen carbonate, purified lanolin, microcrystalline cellulose, dextrin, sodium alginate, methyl cellulose, sodium carboxymethylcellulose, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate, and Primojel^{®}; and binders such as gelatin, Arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch, hydroxypropyl cellulose, hydroxypropyl methylcellulose, polyvinyl alcohol, and polyvinylpyrrolidone may be used, and disintegrants such as hydroxypropyl methylcellulose, corn starch, agar powder, methylcellulose, bentonite, hydroxypropyl starch, sodium carboxymethylcellulose, sodium alginate, calcium carboxymethylcellulose, calcium citrate, sodium lauryl sulfate, silicic anhydride, 1-hydroxypropylcellulose, dextran, ion-exchange resin, polyvinyl acetate, formaldehyde-treated casein and gelatin, alginic acid, amylose, guar gum, sodium bicarbonate, polyvinylpyrrolidone, calcium phosphate, gelled starch, Arabic gum, amylopectin, pectin, sodium polyphosphate, ethyl cellulose, white sugar, magnesium aluminum silicate, a di-sorbitol solution, and light anhydrous silicic acid; and lubricants such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid may be used.

As additives of liquids according to the present invention, water, dilute hydrochloric acid, dilute sulfuric acid, sodium citrate, monostearic acid sucrose, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamine, polyvinylpyrrolidone, ethylcellulose, and sodium carboxymethylcellulose may be used.

In syrups according to the present invention, a white sugar solution, other sugars or sweeteners, and the like may be used, and as necessary, a fragrance, a colorant, a preservative, a stabilizer, a suspending agent, an emulsifier, a viscous agent, or the like may be used.

In emulsions according to the present invention, purified water may be used, and as necessary, an emulsifier, a preservative, a stabilizer, a fragrance, or the like may be used.

In suspensions according to the present invention, suspending agents such as acacia, tragacanth, methylcellulose, carboxymethylcellulose, sodium carboxymethylcellulose, microcrystalline cellulose, sodium alginate, hydroxypropyl methylcellulose (HPMC), HPMC 1828, HPMC 2906, HPMC 2910, and the like may be used, and as necessary, a surfactant, a preservative, a stabilizer, a colorant, and a fragrance may be used.

Injections according to the present invention may include: solvents such as distilled water for injection, a 0.9% sodium chloride solution, Ringer's solution, a dextrose solution, a dextrose+sodium chloride solution, PEG, lactated Ringer's solution, ethanol, propylene glycol, non-volatile oil-sesame oil, cottonseed oil, peanut oil, soybean oil, corn oil, ethyl oleate, isopropyl myristate, and benzene benzoate; cosolvents such as sodium benzoate, sodium salicylate, sodium acetate, urea, urethane, monoethylacetamide, butazolidine, propylene glycol, the Tween series, amide nicotinate, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite (NaHSO3) carbon dioxide gas, sodium metabisulfite (Na2S2O5), sodium sulfite (Na2SO3), nitrogen gas (N2), and ethylenediamine tetraacetic acid; sulfating agents such as 0.1% sodium bisulfide, sodium formaldehyde sulfoxylate, thiourea, disodium ethylenediaminetetraacetate, and acetone sodium bisulfite; a pain relief agent such as benzyl alcohol, chlorobutanol, procaine hydrochloride, glucose, and calcium gluconate; and suspending agents such as sodium CMC, sodium alginate, Tween 80, and aluminum monostearate.

In suppositories according to the present invention, bases such as cacao butter, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter + cholesterol, lecithin, lanette wax, glycerol monostearate, Tween or span, imhausen, monolan(propylene glycol monostearate), glycerin, Adeps solidus, buytyrum Tego-G, cebes Pharma 16, hexalide base 95, cotomar, Hydrokote SP, S-70-XXA, S-70-XX75(S-70-XX95), Hydrokote 25, Hydrokote 711, idropostal, massa estrarium (A, AS, B, C, D, E, I, T), masa-MF, masupol, masupol-15, neosuppostal-N, paramount-B, supposiro OSI, OSIX, A, B, C, D, H, L, suppository base IV types AB, B, A, BC, BBG, E, BGF, C, D, 299, suppostal N, Es, Wecoby W, R, S, M, Fs, and tegester triglyceride matter (TG-95, MA, 57) may be used.

Solid preparations for oral administration include tablets, pills, powders, granules, capsules, and the like, and such solid preparations are formulated by mixing the composition with at least one excipient, e.g., starch, calcium carbonate, sucrose, lactose, gelatin, and the like. In addition to simple excipients, lubricants such as magnesium stearate and talc are also used. Examples of liquid preparations for oral administration include suspensions, liquids for internal use, emulsions, syrups, and the like, and these liquid preparations may include, in addition to simple commonly used diluents, such as water and liquid paraffin, various types of excipients, for example, a wetting agent, a sweetener, a fragrance, a preservative, and the like. Preparations for parenteral administration include an aqueous sterile solution, a non-aqueous solvent, a suspension, an emulsion, a freeze-dried preparation, and a suppository. Non-limiting examples of the non-aqueous solvent and the suspension include propylene glycol, polyethylene glycol, a vegetable oil such as olive oil, and an injectable ester such as ethyl oleate.

The pharmaceutical composition according to the present invention is administered in a pharmaceutically effective amount. In the present invention, "the pharmaceutically effective amount" refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields.

The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

The pharmaceutical composition of the present invention may be administered to a subject via various routes. All administration methods can be predicted, and the pharmaceutical composition may be administered via, for example, oral administration, subcutaneous injection, intraperitoneal injection, intravenous injection, intramuscular injection, intrathecal (space around the spinal cord) injection, sublingual administration, administration via the buccal mucosa, intrarectal insertion, intravaginal insertion, ocular administration, intra-aural administration, intranasal administration, inhalation, spraying via the mouth or nose, transdermal administration, percutaneous administration, or the like.

The pharmaceutical composition of the present invention is determined depending on the type of a drug, which is an active ingredient, along with various related factors such as a disease to be treated, administration route, the age, gender, and body weight of a patient, and the severity of diseases. Specifically, the effective amount of the composition according to the present invention may vary depending on the age, sex, and body weight of a patient, and generally, 0.001 to 150 mg per 1 kg of body weight, preferably 0.01 to 100 mg, may be administered daily or every other day, or may be administered one to three times per day. However, the dosage may be increased or decreased depending on the administration route, severity of the disease, sex, body weight, age, and the like, and therefore, the above dosage does not limit the scope of the present invention in any way.

As used herein, the "subject" refers to a subject in need of treatment of a disease, and more specifically, refers to a mammal such as a human or a non-human primate, a mouse, a rat, a dog, a cat, a horse, and a cow.

As used herein, the "administration" refers to providing a subject with a predetermined composition of the present invention by using an arbitrary appropriate method.

The term "prevention" as used herein means all actions that inhibit or delay the onset of a target disease. The term "treatment" as used herein means all actions that alleviate or beneficially change a target disease and abnormal metabolic symptoms caused thereby via administration of the pharmaceutical composition according to the present invention. The term "alleviation" as used herein means all actions that reduce the degree of parameters related to a target disease, e.g., symptoms via administration of the composition according to the present invention.

Additionally, the present invention provides a kit for preventing or treating head and neck cancer, comprising the pharmaceutical composition according to the present invention and instructions.

The kit according to the present invention may include, in addition to the compound and the anti-cancer agent, other components, compositions, solutions, devices, and the like that are generally required for the prevention or treatment of head and neck cancer without limitation, and in particular, may include instructions indicating appropriate use and storage of the compound according to the present invention. All components included in the kit may be used one or more times without limitation, there is no limitation on the order of using each material, and the application of each material may be performed simultaneously or at different times.

Additionally, the kit of the present invention may include a container in addition to the compound, the anti-cancer agent, and the like. The container may serve to package the components and may also serve to store and fix the components. The container may take, for example, a form such as a bottle, tub, sachet, envelope, tube, ampoule, or the like, and may be formed partially or entirely from plastic, glass, paper, foil, wax, or the like. The container may be equipped with a completely or partially removable stopper, which may initially be part of the container or may be attached to the container by mechanical, adhesive, or other means, and may also be equipped with a stopper through which the contents may be accessed by a syringe needle. The kit may include an external package, and the external package may include instructions regarding the use of the components, but is not limited thereto.

As used herein, the term "active ingredient" refers to a component that exhibits a desired activity alone or may exhibit a desired activity together with a carrier or the like that itself has no activity.

The terms or words used in the present specification and claims should not be construed as being limited to ordinary or dictionary meanings, and should be construed as meanings and concepts consistent with the technical spirit of the present invention based on the principle that an inventor may appropriately define the concept of terms in order to describe his or her own invention in the best manner.

### [Modes of the Invention]

Hereinafter, preferred examples are presented to help understand the present invention. However, the following examples are provided only to help understand the present invention more easily, and the contents of the present invention are not limited by the following examples.

### [Examples]

### Example 1. Synthesis of Compound AON-MG23 of the present invention

### <Step 1> Synthesis of N-cyclopropyl-N-(2-nitrophenyl)methanesulfonamide

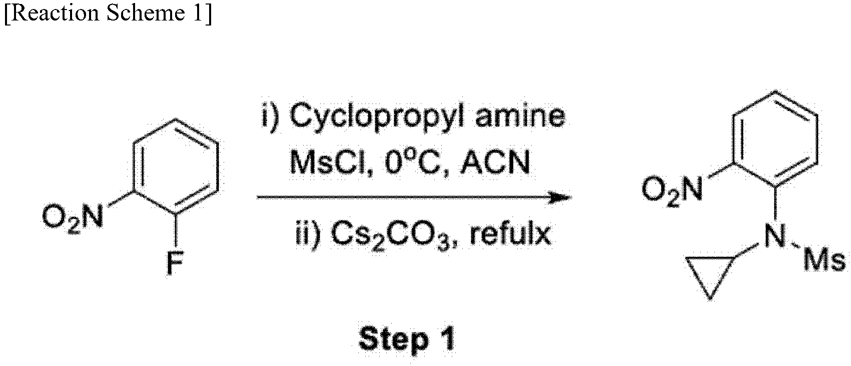

1-Fluoro-2-nitrobenzene (1.00 eq.) and cyclopropylamine (1.00 eq.) were dissolved in acetonitrile (ACN), methanesulfonyl chloride (MsCl, 1.0 eq.) was slowly added at 0 °C, and the resulting mixture was stirred at the same temperature for one hour. Thereafter, cesium carbonate (Cs₂CO₃, 5.00 eq.) was added at the same temperature, and then the resulting mixture was stirred under reflux overnight. After the reaction was complete, the temperature was lowered to room temperature, and extraction was performed using ethyl acetate and water. Anhydrous sodium sulfate was added to the organic layer and stirred, the resulting mixture was filtered using a filter, and the filtrate was concentrated under reduced pressure. The concentrated residue was purified by column chromatography (hexane:ethyl acetate = 3:1) to synthesize N-cyclopropyl-N-(2-nitrophenyl)methanesulfonamide as a pale yellow solid, and the yield was 36%.

### <Step 2> Synthesis of N-(2-aminophenyl)-N-cyclopropylmethanesulfonamide

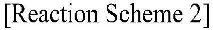

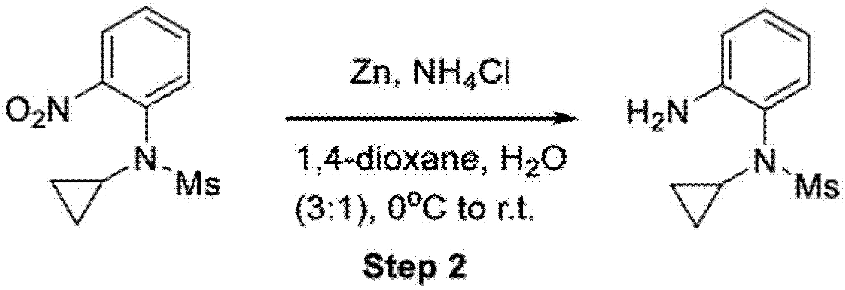

N-cyclopropyl-N-(2-nitrophenyl)methanesulfonamide (1.00 eq.) synthesized in Step 1 was added to 1,4-dioxane and water (3:1), and the resulting mixture was stirred. Thereafter, the reactor was cooled to 0 °C, and zinc (Zn, 10.0 eq.) and ammonium chloride (NH₄Cl, 10.0 eq.) were added. Thereafter, the temperature was gradually increased while stirring for four hours. After the reaction was complete, the mixture was filtered using Celite, and the filtrate was extracted with ethyl acetate and water. Anhydrous sodium sulfate was added to the organic layer and stirred, the resulting mixture was filtered using a filter, and the filtrate was concentrated under reduced pressure. The concentrated residue was used in Step 3 without a separate purification process.

### <Step 3> Synthesis of N-cyclopropyl-N-(2-((2,5-dichloropyrimidine-4-yl)amino)phenyl)methanesulfonamide

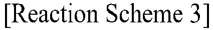

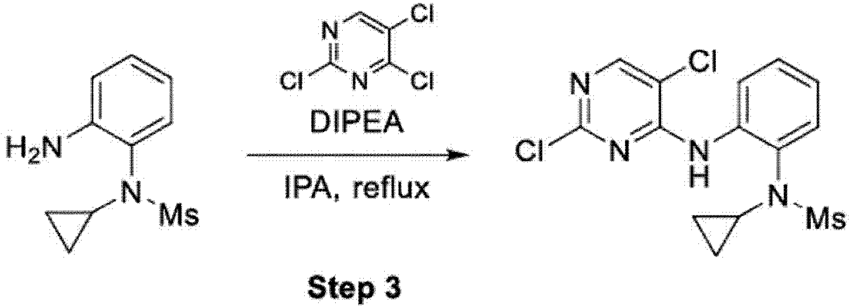

N-(2-aminophenyl)-N-cyclopropylmethanesulfonamide (1.00 eq.) synthesized in Step 2 was added to isopropyl alcohol (IPA), and 2,4,5-trichloropyrimidine (1.1 eq.) and N,N-diisopropylethylamine (DIPEA, 2.5 eq.) were added at room temperature. Thereafter, the resulting mixture was stirred overnight under reflux. After the reaction was complete, the mixture was evaporated under reduced pressure and extracted using water and dichloromethane. The organic layer was washed with 2 N hydrochloric acid (HCl), anhydrous sodium sulfate was added to the organic layer, and the resulting mixture was stirred. Thereafter, the mixture was filtered using a filter, and the filtrate was concentrated under reduced pressure. The concentrated residue was purified by column chromatography (hexane:ethyl acetate = 3:1) to synthesize N-cyclopropyl-N-(2-((2,5-dichloropyrimidin-4-yl)amino)phenyl)methanesulfonamide as a pale yellow solid, and the yield was 58.9%.

### <Step 4> Synthesis of N-(2-((5-chloro-2-((4-(4-(dimethylamino)piperidine-1-yl)-2-methoxyphenyl)amino)pyrimidine-4-yl)amino)phenyl)-N-cyclopropylmethanesulfonamide

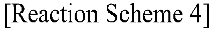

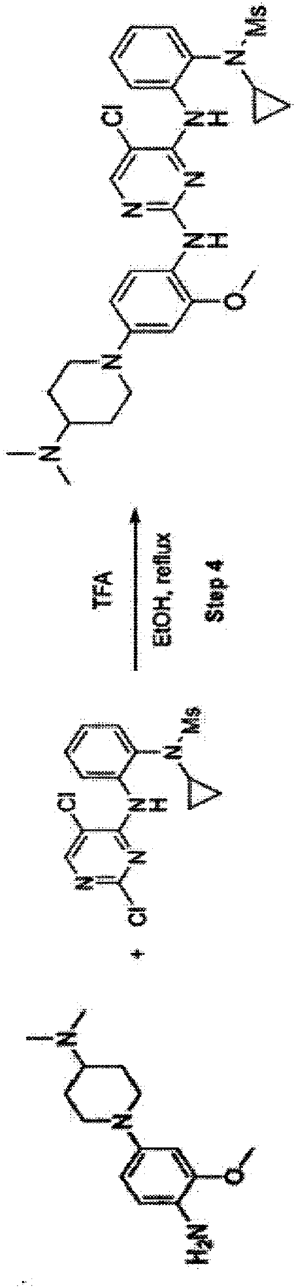

N-cyclopropyl-N-(2-((2,5-dichloropyrimidine-4-yl)amino)phenyl)methanesulfonamide (1.00 eq.) synthesized in Step 3 was added to ethanol (EtOH), 1-(4-amino-3-methoxyphenyl)-N,N-dimethylpiperidin-4-amine (1.00 eq.) and trifluoroacetic acid (TFA, 1.95 eq.) were added at room temperature, and the resulting mixture was stirred overnight under reflux. After the reaction was complete, the mixture was neutralized with a 1 N sodium hydroxide (NaOH) solution and extracted with water and ethyl acetate. Anhydrous sodium sulfate was added to the organic layer, the resulting mixture was mixed, stirred, and then filtered using a filter, and the filtrate was concentrated under reduced pressure. The concentrated residue was purified by column chromatography (hexane:ethyl acetate = 3:1) to synthesize N-(2-((5-chloro-2-((4-(4-(dimethylamino)piperidin-1-yl)-2-methoxyphenyl)amino)pyrimidin-4-yl)amino)phenyl)-N-cyclopropylmethanesulfonamide (AON-MG23) as a yellow solid, and the yield was 25%.

¹H nuclear magnetic resonance (NMR) (500 MHz, dimethyl sulfoxide (DMSO)-d6) δ 8.33 (s, 1H), 8.19 (s, 1H), 8.09 (s, 1H), 7.99 (s, 1H), 7.62 (dd, J = 7.8, 1.6 Hz, 1H), 7.35 (d, J = 8.7 Hz, 1H), 7.22 (d, J = 8.1 Hz, 1H), 7.15 (td, J = 7.6, 1.6 Hz, 1H), 6.63 (d, J = 2.5 Hz, 1H), 6.48 (dd, J = 8.7, 2.5 Hz, 1H), 3.75 (s, 3H), 3.72 (d, J = 12.2 Hz, 2H), 3.23 (m, 4H), 2.68 (td, J = 12.1, 2.5 Hz, 2H), 2.22 (s, 6H), 1.86 (d, J = 12.4 Hz, 2H), 1.51 (qd, J = 12.0, 3.9 Hz, 2H), 1.04 - 0.92 (m, 2H), 0.55 - 0.49 (m, 1H), 0.17 - 0.14 (m, 1H). High-resolution mass spectrometry (HRMS): 585.2294, cal: 585.2289.

### Example 2. Confirmation of proliferation inhibitory effect of compound AON-MG23 of the present invention on head and neck cancer cells

FaDu cells and squamous cell carcinoma-25 (SCC-25) cells, which are human head and neck cancer cell lines, were seeded in 96-well plates at a density of 1 x 10⁵ cells/well and cultured for 24 hours. Thereafter, the cells were treated with compound AON-MG23 of the present invention at various concentrations and cultured for 48 hours. The cells were treated with the compound of the present invention at various concentrations of 0 µM, 0.5 µM, 1 µM, 2 µM, and 4 µM. 10 µL of cell counting kit (CCK) reagent was added and allowed to react for one hour, and then the absorbance was measured at 450 nm.

As a result, as shown in FIG. 1, it was confirmed that compound AON-MG23 of the present invention inhibited the proliferation of head and neck cancer cells.

### Example 3. Confirmation of proliferation inhibitory effect of compound AON-MG23 of the present invention on head and neck cancer cells

To confirm the proliferation inhibitory effect of compound AON-MG23 of the present invention on head and neck cancer cells, a clonogenic assay was conducted. Specifically, head and neck cancer cell line FaDu cells were plated in a 6-well cell culture plate at a density of 250 cells per well. On the next day, the cell culture medium was removed and replaced with a cell culture medium containing compound AON-MG23 of the present invention at concentrations of 0 µM, 0.125 µM, 0.25 µM, 0.5 µM, and 1 µM. After culturing the cells for nine days, the resulting cell colonies were stained and photographed, and then the cell colonies were counted and analyzed using Image J software.

As a result, as shown in FIG. 2, it was confirmed that clonogenesis of the FaDu cells was inhibited after treatment with compound AON-MG23 of the present invention.

### Example 4. Confirmation of inhibitory effect of compound AON-MG23 of the present invention on cell migration in head and neck cancer cells

Human head and neck cancer cell lines, FaDu cells and SCC-25 cells, were seeded in SPLScar Block plates (SPL) at a density of 7.2 x 10⁴/well and cultured for 24 hours. After 24 hours, the blocks were removed, and at the same time, compound AON-MG23 of the present invention was applied at various concentrations. Thereafter, cells were cultured in a CO₂ incubator at 37 °C for 17 hours. Specifically, the FaDu cells were treated with compound AON-MG23 of the present invention at concentrations of 0 µM, 0.25 µM, 0.5 µM, 1 µM, and 2 µM, and the SCC-25 cells were also treated at concentrations of 0 µM, 0.25 µM, 0.5 µM, 1 µM, and 2 µM. Photographs were taken before and after each culture, and the migration distance of the cells was measured using Image J software. Based on the results, the inhibition of the cell migration was analyzed.

As a result, as shown in FIGS. 3A and 3B, it was confirmed that when cells were treated with compound AON-MG23 of the present invention at a concentration of 2 µM, the migration of the FaDu cells was inhibited by 80% or more compared to the control group, and when cells were treated with the compound at a concentration of 1 µM, the migration of the SCC-25 cells was inhibited by about 90% compared to the control group.

### Example 5. Confirmation of inhibitory effect of compound AON-MG23 of the present invention on head and neck cancer cell metastasis

Human head and neck cancer cell lines, FaDu cells and SCC-25 cells, were seeded at a density of 6 x 10⁴ cells/well on top of filters coated with Matrigel (Transwell invasion chamber, Corning). Thereafter, the FaDu cells were treated with compound AON-MG23 of the present invention at concentrations of 0 µM, 0.25 µM, 0.5 µM, 1 µM, and 2 µM, and the SCC-25 cells were also treated with the compound at concentrations of 0 µM, 0.25 µM, 0.5 µM, 1 µM, and 2 µM. Cells were cultured in a CO₂ in an incubator at 37 °C for 24 hours. After culture, cell fixation and staining were performed using the Diff-Quick stain Kit (Sysmex, Kobe, Japan). The stained cells were imaged under a microscope, and the cells were counted and statistically analyzed using Image J software.

As a result, as shown in FIGS. 4A and 4B, it was confirmed that the groups treated with compound AON-MG23 of the present invention exhibited a reduced degree of penetration into Matrigel compared to the untreated control group, thereby indicating that the metastatic potential of the cancer cells was inhibited.

### Example 6. Confirmation of the effect of compound AON-MG23 of the present invention on the expression of anoctamin-1 (ANO1) and epidermal growth factor receptor (EGFR) proteins in head and neck cancer cell lines

Human head and neck cancer cell lines, FaDu cells and SCC-25 cells, were seeded in 60 mm dishes at a density of 5 × 10⁵ cells and cultured for 24 hours. Thereafter, the FaDu cells were treated with compound AON-MG23 of the present invention at concentrations of 0 µM, 0.25 µM, 0.5 µM, 1 µM, 2 µM, and 4 µM, and the SCC-25 cells were treated with the compound at concentrations of 0 µM, 0.25 µM, 0.5 µM, 1 µM, and 2 µM. The cells were cultured for 48 hours. After culture, the cells were recovered, and the recovered cells were lysed to perform Western blot. As antibodies used for Western blot, an anti-ANO1 antibody (Abcam, ab53212), an anti-EGFR antibody (Cell Signaling, #4267S), and an anti-glyceraldehyde 3-phosphate dehydrogenase (GAPDH) antibody (Santacruz, #SC-5286) were used. Protein concentration was quantified using the bovine serum albumin (BSA) method (Protein Quantitation Assay, BSA assay) (Pierce, Cat.23225). After lysing the cells, the proteins obtained therefrom were separated by molecular weight using 10% sodium dodecyl sulfate-polyacrylamide gel electrophoresis (SDS-PAGE) with equal amounts (20 µg). After transferring the proteins to a polyvinylidene fluoride (PVDF) membrane (Bio-Rad), the proteins were treated with a 5% blocking buffer (5% skim milk in Tris-buffered saline (TBS) buffer containing 0.1% Tween 20 (TBS-T)) at room temperature for one hour. Subsequently, the membrane was treated with a primary antibody, which was allowed to react at 4 °C for 16 hours. After the reaction was complete, the membrane was washed three times with TBS-T. Subsequently, the membrane was treated with a secondary antibody labeled with horseradish peroxidase at room temperature for one hour and then visualized using an enhanced chemiluminescence (ECL) kit (Bio-Rad). The amount of each protein was quantified and analyzed using Image J software.

As a result, as shown in FIGS. 5A and 5B, it was confirmed that both the ANO1 protein and EGFR protein levels were significantly inhibited in the cells treated with compound AON-MG23 of the present invention compared to the control group and that this protein expression inhibitory effect was dependent on the compound treatment concentration.

### Example 7. Confirmation of drug combination effect of compound AON-MG23 of the present invention on head and neck cancer cells

The combination effect of compound AON-MG23 of the present invention was confirmed using Bliss scoring based on the results of the sulforhodamine B assay (SRB assay). Specifically, human head and neck cancer cell line FaDu cells were plated in a 96-well cell culture plate at a density of 1 x 10³ cells/well. On the next day, in order to confirm the combination effect with compound AON-MG23 of the present invention, a drug, paclitaxel or osimertinib, was diluted to concentrations for testing, and cells were treated with the diluted drug and then cultured for five days. After five days of co-treatment, the culture medium mixed with the drug was removed, and 10% trichloroacetic acid (TCA) was added to each well for fixation at 4 °C for one hour. After removing the TCA, the 96-well plate was washed five times with water and dried at room temperature for one hour. Thereafter, each well was treated with a 0.4% sulforhodamine B (SRB) solution (SRB in 1% acetic acid) to stain the cells at room temperature for 30 minutes. After removing the 0.4% SRB solution, the plate was washed five times with 1% acetic acid and dried again for one hour. After drying, 200 µL of 10 mM Tris-base (pH 10.5) was added to each well, and the stained dye was dissolved by stirring at room temperature for 10 minutes. The absorbance of the dissolved solution was measured at 540 nm using a plate reader. The measured absorbance values were compared to the control group and converted into percentages, and the combination effect was analyzed using the percentage data at an analysis website (https://synergyfinder.fimm.fi/). The presence of a combination effect was evaluated according to the following criteria based on relevant literature (Bliss, C. I. (1939). The toxicity of poisons applied jointly. Annals of Applied Biology, 26(3), 585-615.): a score > 0 indicates a synergistic effect; a score = 0 indicates an independent effect; and a score < 0 indicates an antagonistic effect.

As shown in FIGS. 6A and 6B, the Bliss synergy score of compound AON-MG23 of the present invention and paclitaxel in human head and neck cancer cell line FaDu cells was 6.72 points, and the Bliss synergy score of AON-MG23 and osimertinib was 19.25, indicating a high combination effect.

The foregoing description of the present invention is intended for illustrative purposes, and it will be understood by those skilled in the art that various modifications can be made thereto in other specific forms without departing from the technical spirit or essential features of the present invention. Therefore, the embodiments described above should be understood as illustrative in all respects and not restrictive.

### [Industrial Applicability]

The novel compound of the present invention not only significantly inhibits the growth, proliferation, migration, and metastasis of head and neck cancer cells, but also may simultaneously inhibit ANO1 and EGFR in head and neck cancer cells, thereby exerting a stronger anti-cancer effect through dual inhibition of the two proteins. Additionally, when used in combination with an anti-cancer agent or the like, the compound may further enhance the efficacy of the anti-cancer agent and suppress cancer resistance. Therefore, the compound may be used as a dual-target anti-cancer agent for ANO1 and EGFR on its own, and may also be utilized as a combination preparation with an anti-cancer agent, and thus may be widely utilized in the field of prevention and treatment of head and neck cancer, and therefore has industrial applicability.

## Claims

1. A pharmaceutical composition for preventing or treating head and neck cancer, comprising, as an active ingredient, a compound represented by Chemical Formula 1 or a pharmaceutically acceptable salt thereof:

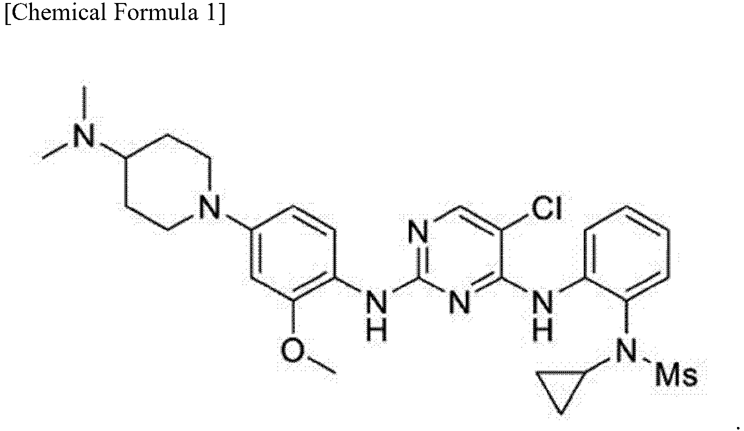

2. The pharmaceutical composition of claim 1, wherein the compound or a pharmaceutically acceptable salt thereof simultaneously inhibits epidermal growth factor receptor (EGFR) and anoctamin-1 (ANO1).

3. The pharmaceutical composition of claim 1, wherein the compound or a pharmaceutically acceptable salt thereof satisfies one or more properties selected from the group consisting of:
(a) inhibiting the proliferation or growth of cancer cells;
(b) inhibiting the migration of cancer cells;
(c) inhibiting the metastasis of cancer; and
(d) inhibiting the resistance of cancer to anti-cancer agents.

4. The pharmaceutical composition of claim 3, wherein the anti-cancer agent is one or more selected from the group consisting of a taxane-based chemotherapy agent and a targeted anti-cancer agent against EGFR.

5. The pharmaceutical composition of claim 4, wherein the taxane-based chemotherapy agent is one or more selected from the group consisting of docetaxel, cabazitaxel, and paclitaxel.

6. The pharmaceutical composition of claim 4, wherein targeted anti-cancer agent against EGFR is one or more selected from the group consisting of cetuximab, panitumumab, osimertinib, gefitinib, afatinib, erlotinib, and lazertinib.

7. The pharmaceutical composition of claim 1, wherein the head and neck cancer is one or more selected from the group consisting of nasal cavity cancer, paranasal sinus cancer, oral cancer, nasopharyngeal cancer, oropharyngeal cancer, hypopharyngeal cancer, laryngeal cancer, cervical esophageal cancer, salivary gland cancer, salivary gland tumors, tongue cancer, thyroid cancer, and tonsil cancer.

8. The pharmaceutical composition of claim 1, wherein the pharmaceutical composition is administered in combination with a taxane-based chemotherapy agent or a targeted anti-cancer agent against EGFR.

9. The pharmaceutical composition of claim 8, wherein the pharmaceutical composition is administered simultaneously, separately, or sequentially with the anti-cancer agent.

10. A pharmaceutical composition for preventing or treating head and neck cancer, comprising as an active ingredient:
(a) a compound represented by Chemical Formula 1 or a pharmaceutically acceptable salt thereof; and
(b) one or more anti-cancer agents selected from the group consisting of a taxane-based chemotherapy agent and a targeted anti-cancer agent against epidermal growth factor receptor (EGFR):

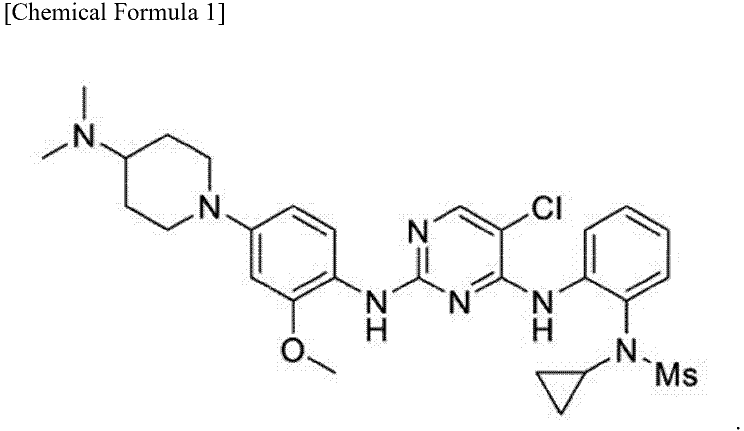

11. The pharmaceutical composition of claim 10, wherein the compound or a pharmaceutically acceptable salt thereof inhibits the resistance of cancer cells to the anti-cancer agent.

12. The pharmaceutical composition of claim 10, wherein the composition is in the form of a mixture mixing the compound or a pharmaceutically acceptable salt thereof; and the anti-cancer agent.

13. The pharmaceutical composition of claim 10, wherein the composition is in a form in which the compound or a pharmaceutically acceptable salt thereof; and the anti-cancer agent are each formulated to be administered simultaneously, separately, or sequentially.

14. A pharmaceutical composition for enhancing an anti-cancer effect of an anti-cancer agent on head and neck cancer, comprising, as an active ingredient, a compound represented by Chemical Formula 1 or a pharmaceutically acceptable salt thereof:

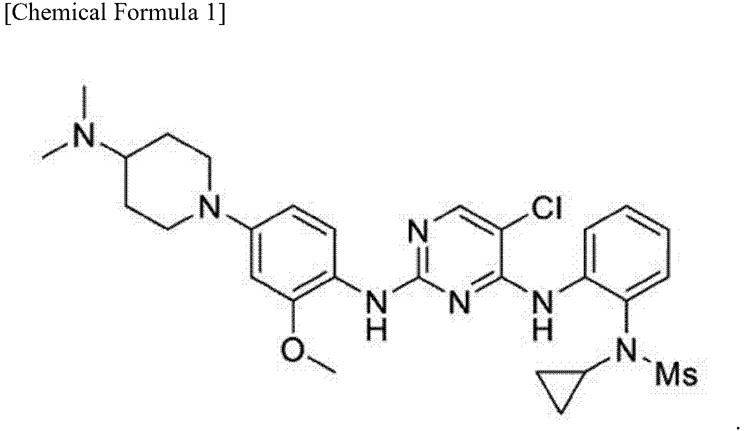

15. The pharmaceutical composition of claim 14, wherein the anti-cancer agent is one or more selected from the group consisting of a taxane-based chemotherapy agent and a targeted anti-cancer agent against epidermal growth factor receptor (EGFR).

16. The pharmaceutical composition of claim 14, wherein the taxane-based chemotherapy agent is one or more selected from the group consisting of docetaxel, cabazitaxel, and paclitaxel.

17. The pharmaceutical composition of claim 14, wherein the targeted anti-cancer agent against EGFR is one or more selected from the group consisting of cetuximab, panitumumab, osimertinib, gefitinib, afatinib, erlotinib, and lazertinib.

18. The pharmaceutical composition of claim 14, wherein the pharmaceutical composition is administered simultaneously, separately, or sequentially with the anti-cancer agent.

19. A method of preventing, ameliorating, or treating head and neck cancer, comprising a step of administering a compound represented by Chemical Formula 1 or a pharmaceutically acceptable salt thereof; or a composition comprising the same, in a pharmaceutically effective amount to a subject in need thereof:

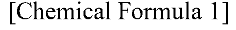

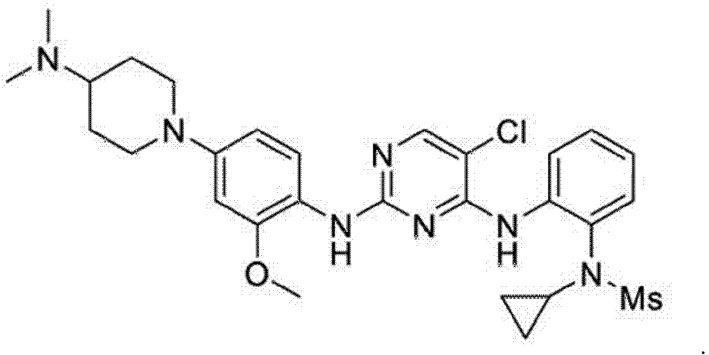

20. A use of a compound represented by Chemical Formula 1 or a pharmaceutically acceptable salt thereof; or a composition comprising the same for preventing, ameliorating, or treating head and neck cancer:

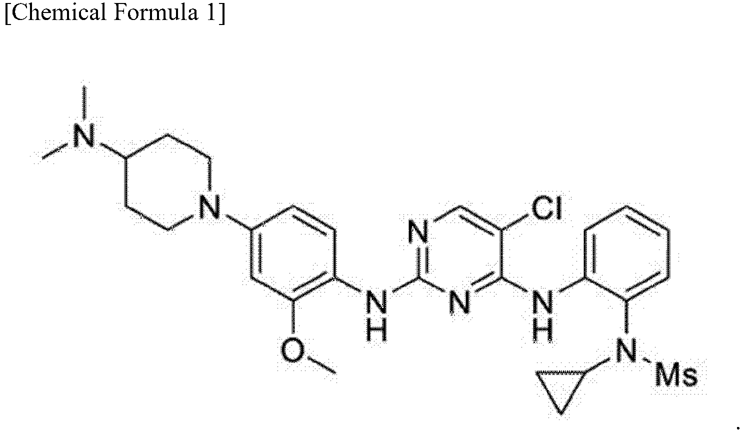

21. A use of a compound represented by Chemical Formula 1 or a pharmaceutically acceptable salt thereof; or a composition comprising the same for preparing a preparation for preventing, ameliorating, or treating head and neck cancer:

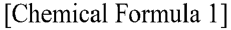

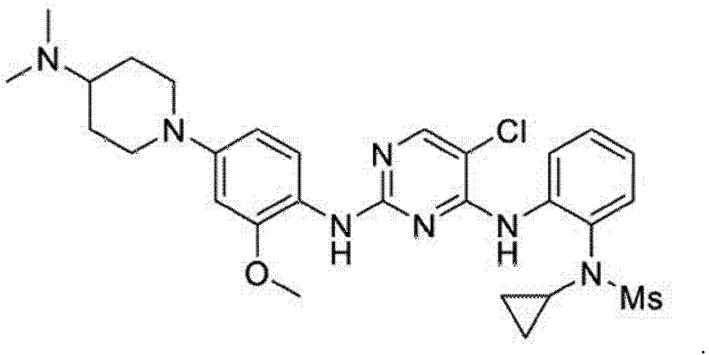

22. A method of enhancing an anti-cancer effect of an anti-cancer agent on head and neck cancer, comprising a step of administering a compound represented by Chemical Formula 1 or a pharmaceutically acceptable salt thereof; or a composition comprising the same, in a pharmaceutically effective amount to a subject in need thereof:

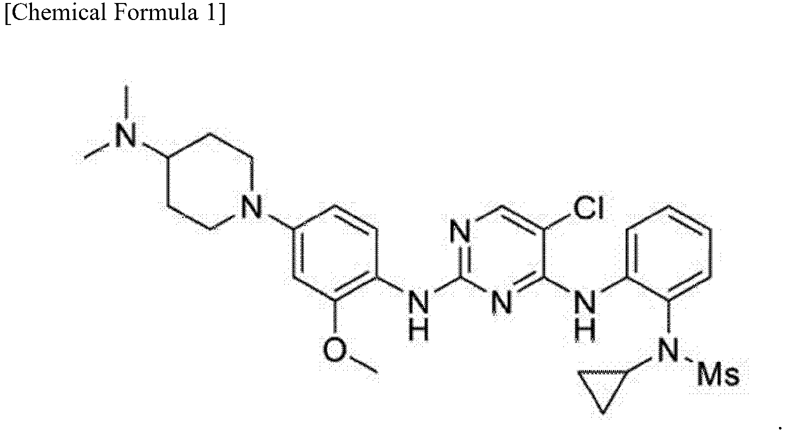

23. A use of a compound represented by Chemical Formula 1 or a pharmaceutically acceptable salt thereof; or a composition comprising the same for enhancing an anti-cancer effect of an anti-cancer agent on head and neck cancer:

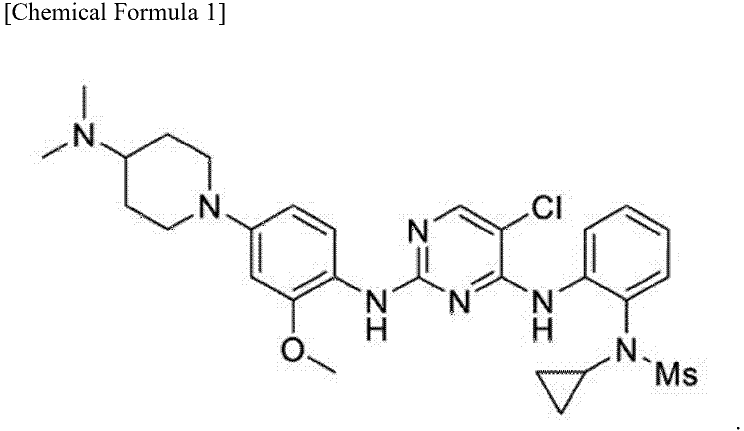

24. A use of a compound represented by Chemical Formula 1 or a pharmaceutically acceptable salt thereof; or a composition comprising the same for preparing a preparation for enhancing an anti-cancer effect of an anti-cancer agent on head and neck cancer:

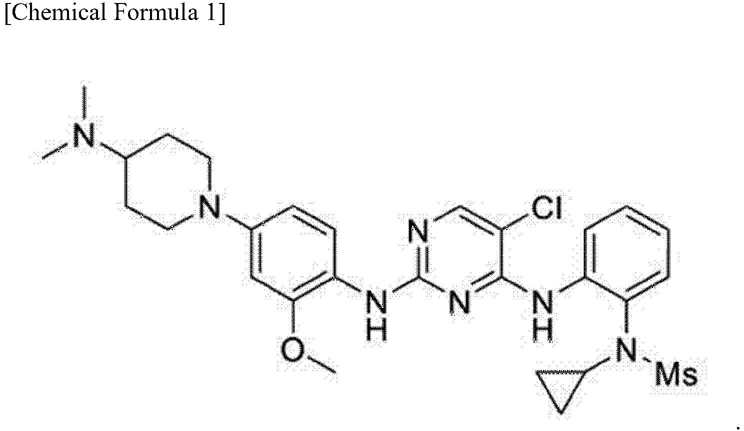

25. A method of preventing, ameliorating, or treating head and neck cancer, comprising a step of administering (a) a compound represented by Chemical Formula 1 or a pharmaceutically acceptable salt thereof; and (b) one or more anti-cancer agents selected from the group consisting of a taxane-based chemotherapy agent and a targeted anti-cancer agent against epidermal growth factor receptor (EGFR); or a composition comprising the same, in a pharmaceutically effective amount to a subject in need thereof:

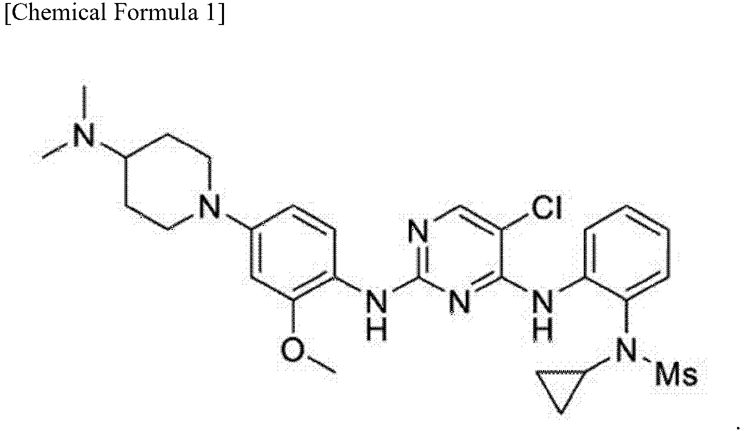

26. A use of (a) a compound represented by Chemical Formula 1 or a pharmaceutically acceptable salt thereof; and (b) one or more anti-cancer agents selected from the group consisting of a taxane-based chemotherapy agent and a targeted anti-cancer agent against epidermal growth factor receptor (EGFR); or a composition comprising the same for preventing, ameliorating, or treating head and neck cancer:

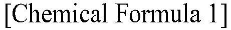

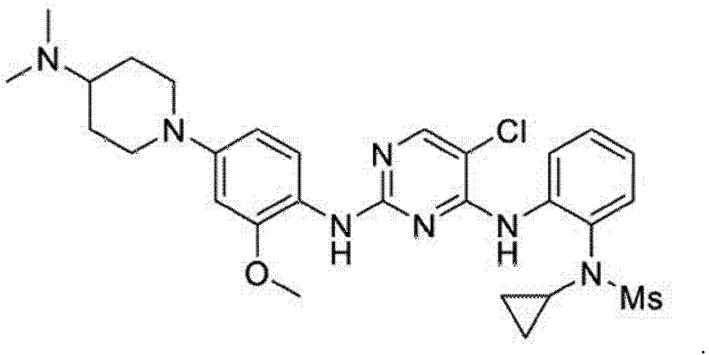

27. A use of (a) a compound represented by Chemical Formula 1 or a pharmaceutically acceptable salt thereof; and (b) one or more anti-cancer agents selected from the group consisting of a taxane-based chemotherapy agent and a targeted anti-cancer agent against epidermal growth factor receptor (EGFR); or a composition comprising the same for preparing a preparation for preventing, ameliorating, or treating head and neck cancer:
